# EUROPEAN PATENT APPLICATION

(11) **EP 3 812 769 A1**
(43) Date of publication of application: **28.04.2021**
(21) Application number: 19204750.4
(22) Date of filing: 23.10.2019
(51) Int. Cl.: G01N 33/68

(54) **A METHOD FOR IN VITRO DIAGNOSIS OF RENAL GLOMERULAR DISEASE OR FOR MONITORING THE PROGRESSION OF RENAL GLOMERULAR DISEASE**

(71) Applicant: Unicyte EV AG, 6370 Oberdorf (CH)
(72) Inventor: BUSSOLATI, Benedetta, 10126 TORINO (IT); CAMUSSI, Giovanni, 10132 TORINO (IT); DIMUCCIO, Veronica, 10128 TORINO (IT)
(74) Representative: Rimini, Rebecca

(57) **Abstract**

There is disclosed an in vitro method of diagnosing or monitoring the progression of renal glomerular disease in a subject. The method of the invention is based on the determination of the amount of extracellular vesicles (EVs) expressing prominin-1 in a biological fluid sample from the subject, wherein the amount of said EVs below a predetermined threshold value is indicative of renal glomerular disease or of progression of renal glomerular disease. Preferably, the biological fluid is urine.

## Description

The present invention relates to the field of diagnosis of kidney diseases, more specifically renal glomerular diseases.

Renal glomeruli excrete urinary substances and excess water as an ultrafiltrate into the urine by selectively filtering the blood. Any damage to the glomeruli disrupts the filtration process and results in the appearance of blood components (proteins and red blood cells) in the urine. A number of different conditions can result in glomerular disease. In a high percentage of cases, glomerular damage is caused by immune-mediated processes, which often lead to glomerulonephritis. Non-inflammatory causes, such as metabolic disease (e.g., diabetes, amyloidosis), can also result in significant damage to the glomeruli. The pathophysiology of glomerular diseases is complex; most patients present with either nephritic syndrome (low-level proteinuria, microhematuria, oliguria, and hypertension) or nephrotic syndrome (high-level proteinuria and generalized edema). Most forms of glomerular disease progress gradually to chronic kidney disease (CKD), often having an insidious and asymptomatic course, which determines diagnostic delay, contributing to a poorer kidney and patient's clinical survival. Chronic kidney disease (CKD) is the slow, gradual loss of kidney function through mild, moderate, and severe chronic kidney failure. Therefore, strategies to prevent irreversible kidney damage require effective methods of early diagnosing in the glomerular disease process.

The assessment and diagnosis of renal function during acute and chronic pathologies affecting the renal compartments is at present mainly based on levels of retention markers such as creatinine and urea. However, creatinine-based estimates of renal function are not reliable in patients with changing serum creatinine levels (as occurs in many hospitalized patients) as well as in patients with acute kidney injury. Moreover, serum creatinine depend on a plurality of different factors such as muscle mass, body size, sex, race, age and medications that affect excretion of creatinine (e.g., trimethoprim).

In addition to creatinine, alternative blood and urinary biomarkers are appearing as precious tools that can refer not only to the degree of renal damage but also to the pathophysiological state of the kidney. In particular, urine detection markers already used in the clinical practice, such as neutrophil gelatinase-associated lipocalin (NGAL) and Cystatin C, appear as the most promising ones as they directly derive from the renal compartment and the urinary tract cells (Holzscheiter L, et al. "NGAL, L-FABP, and KIM-1 in comparison to established markers of renal dysfunction". Clin. Chem. Lab. Med. (2014); 52(4):537-46. doi: 10.1515/cclm-2013-0693). There are, however, several limitations in connection with the diagnostic performance of Cystatin C. Indeed, serum levels are more variable than for creatinine, and the fact that serum levels can be affected by acute disease (malignancy, infection with human immunodeficiency virus) has left cystatin C without a defined role in clinical medicine. Recent studies have also revealed that immaturity of kidney development and inflammatory status represent important factors restricting the usefulness of NGAL as kidney filtration marker.

Extracellular vesicles (EVs) are round vesicles released in biological fluids by almost all types of cells, and they contain proteins and other molecules directly correlated with the tissue from which they originate. Particularly, urinary EVs (uEVs) carry molecules that are characteristic of the epithelial cells facing the whole length of the urinary tract (Miranda KC, et al. "Nucleic acids within urinary exosomes/microvesicles are potential biomarkers for renal disease". Kidney Int. (2010); 78(2):191-9. doi: 10.1038/ki.2010.106; Pisitkun T, et al. "Identification and proteomic profiling of exosomes in human urine". Proc. Natl. Acad. Sci. (2004); 101(36):13368-73. doi: 10.1073/pnas.0403453101). Indeed, proteomic analysis of uEVs from healthy subject demonstrated the presence of renal proteins, such as uromodulin, aquaporin 1 and aquaporin 2, indicating that the majority of EVs derive from tubular segments. EVs may also play a role in the cell-to-cell communication along the urinary lumen. Indeed, proximal tubule derived EVs were shown to be internalized by distal tubular cells (Gildea JJ, et al. "Exosomal transfer from human renal proximal tubule cells to distal tubule and collecting duct cells". Clin. Biochem. (2014); 47(15):89-94. doi: 10.1016/j.clinbiochem.2014.06.018). Furthermore, the increase of water flow in recipient cells proved the functional transfer of AQP2 from EVs deriving from collecting ducts. During glomerular disease such as diabetes, podocyte-derived EVs may promote fibrosis of tubular cells, driven by a detrimental glomerular-tubular communication (Munkonda MN, et al. Podocyte-derived microparticles promote proximal tubule fibrotic signaling via p38 MAPK and CD36. J. Extracell. Vesicles (2018); 7(1):1432206. doi: 10.1080/20013078.2018.1432206).

The study described in Dimuccio V, et al. (2014) "Urinary CD133+ Extracellular Vesicles Are Decreased in Kidney Transplanted Patients with Slow Graft Function and Vascular Damage", PLoS ONE 9(8): e104490 revealed that the level of uEVs expressing CD133, a tubular EV marker, was reduced in the urine of kidney transplant patients with slow graft function compared to early graft function.

The present invention has the object of providing a method for the diagnosis of renal glomerular disease which overcomes the disadvantages and limitations of the prior art.

More particularly, an object of the present invention is to provide a diagnostic method that allows timely identification of patients affected by renal glomerular disease with a high degree of accuracy, especially at an early stage of the disease, thereby preventing and/or delaying disease progression to CKD and improving patient safety.

It is another object of the present invention to provide a diagnostic method which enables monitoring the progression of renal glomerular disease in order to guide effective clinical care and enhance curative success.

This and other objects are achieved by the in vitro method of diagnosing or monitoring the progression of renal glomerular disease, as defined in the appended claim 1. Preferred embodiments of the method of the invention are defined in the dependent claims.

The appended independent and dependent claims are an integral part of the present description.

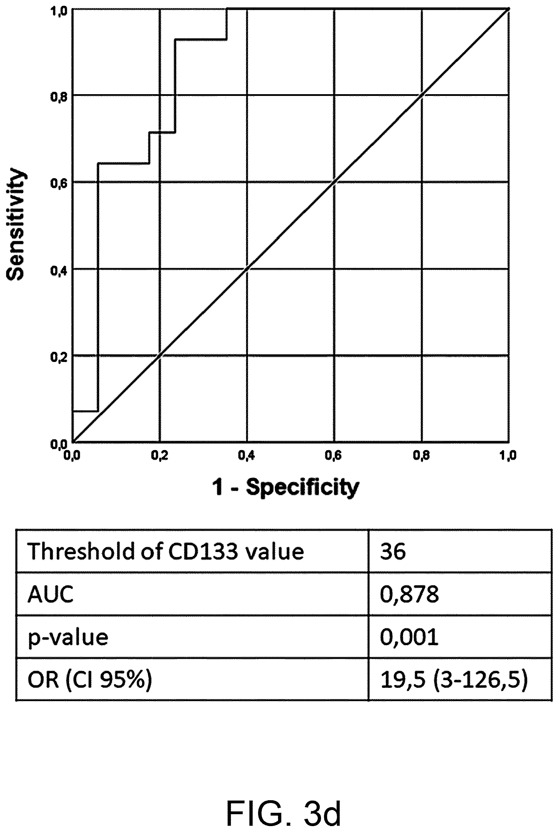

As it will be illustrated in detail in the following examples, with their studies the inventors found that the levels of urinary EVs expressing the adult stem cell marker prominin-1 (Prom1⁺ EVs) significantly declined in the urine of patients affected by acute or chronic kidney disease, particularly a disease involving the glomerular compartment of the kidney, compared to healthy subjects. Further supporting the diagnostic value of Prom1⁺ EVs, the analysis of ROC curve as illustrated in Figure 3d, demonstrated that a test based on the measurement of urinary EVs expressing prominin-1 provides for an accurate discrimination, in terms of both sensitivity and specificity, between healthy subjects and patients with acute or chronic renal glomerular damage. Moreover, as a result of experimental studies carried out by the present inventors on a cohort of patients with progressive kidney disease, the measurement of urinary EVs expressing prominin-1 proved to be a highly reliable tool in achieving a precise disease grading of the tested clinical samples.

Accordingly, an aspect of the present invention is an in vitro method of diagnosing or monitoring the progression of renal glomerular disease in a subject comprising the step of determining in a biological fluid sample from the subject the amount of extracellular vesicles (EVs) expressing prominin-1, wherein the amount of said EVs expressing prominin-1 below a predetermined threshold value is indicative of renal glomerular disease or of progression of renal glomerular disease.

As is known in the art, prominin-1 is a pentaspan transmembrane glycoprotein originally identified in immature hematopoietic cells, and now widely regarded as a marker of normal and cancerous stem cells (Fargeas CA, et al. "Prominin-1 (CD133): from progenitor cells to human diseases". Future Lipidol (2006); 1: 213- 225). Among the various isoforms of prominin-1, CD133 refers to a glycosylated isoform of prominin-1, restricted on hematopoietic stem cells and recognised by AC133 and AC141 mAbs. CD133 molecule represents a marker of a population of tubular cells, intercalated between the other epithelial cells, with the ability to survive to damage and proliferate in response to cell injury (Bussolati B, et al. "CD133+ cells as a therapeutic target for kidney diseases". Expert Opin Ther Targets. (2012) Feb;16(2):157-65).

As used herein, the term "prominin-1" is intended to encompass prominin-1 gene products, and post-translationally processed isoforms, including CD133 as well as prominin1-encoding nucleic acids.

Within the context of the present invention, the term "expressing prominin-1" refers either to the expression of prominin-1 protein or to the expression of prominin1-encoding nucleic acid, for example RNA, as the result of transcription and translation of the prominin-1 gene in the cells giving origin to EVs. It follows that EVs expressing prominin-1 may carry prominin-1 protein and/or the corresponding encoding RNA.

As is further explained in detail below, the method of the invention is characterized by high diagnostic accuracy, i.e. the ability to discriminate between the disease condition and healthy. By performing ROC curve analysis the present inventors found that an amount of Prom1⁺ EVs of from 35% to 40% over the total amount of EVs in the biological fluid sample exhibited the highest power in discriminating between healthy individuals and individuals affected by renal glomerular disease.

Accordingly, in a preferred embodiment, the predetermined threshold value of the method of the invention is comprised within the range of from 35% to 40% of the EVs expressing prominin-1 over the total amount of EVs in the biological fluid sample. Advantageously, in this embodiment, the determination of the relative percentage of prominin-1 EVs over the total EV population makes the method of the invention independent from variation in the total number of EVs among assayed samples.

In a more preferred embodiment, the predetermined threshold value of the method of the invention is 35%, 36%, 37%, 38%, 39% or 40% of the EVs expressing prominin-1 over the total amount of EVs in the biological fluid sample.

As one of skill in the art will appreciate, the expression of prominin-1 in extracellular vesicles may be determined using one of several techniques established in the art, including methods of quantifying protein, such as immunological or activity assay and Western blotting, or methods of quantifying nucleic acid, such as PCR-based techniques, gene expression system, and Northern or Southern blotting techniques.

In one embodiment of the invention, the measurement of the amount of EVs expressing prominin-1 in the test sample is based on the determination of prominin-1 protein. Since prominin-1 is a surface protein on membrane vesicles, this embodiment has the merit of enabling a direct and rapid measurement of Prom1⁺ EVs.

The detection and/or quantification of EVs expressing prominin-1 within the total population of EVs present in the biological sample may be carried out, for example, by applying an immunophenotyping technique, for instance by cytofluorometric analysis using an antibody specifically directed against prominin-1. An exemplary antibody suitable to be employed in the method of the invention is described in EP0953046. Prior to cytofluorometric analysis, the EVs from the biological sample may be absorbed onto a solid surface, such as e.g. a magnetic bead or a non-magnetic bead. A method for capturing extracellular vesicles on a solid surface is disclosed in WO2017/198695, whose content is herein fully incorporated by reference.

According to another embodiment of the method of the invention, the amount of EVs expressing prominin-1 in the test sample is determined by measuring the vesicle content of nucleic acid encoding prominin-1.

By way of example, the quantification of prominin1-encoding RNA in the EVs may be accomplished by a nucleic acid-based amplification technique, more preferably by real-time PCR.

In a preferred embodiment, the EVs content of RNA encoding prominin-1 as assessed by real-time PCR is measured as a threshold cycle (Ct) value.

Typically, nucleic acid quantification by real-time PCR relies on plotting amplification signal, for example fluorescence, against the number of cycles on a logarithmic scale. As used herein, the term "Ct value" refers to the number of PCR cycles required for the amplification signal to reach an intensity above the background level during the exponential phase of the nucleic acid amplification reaction. Consequently, the Ct value is inversely related to the amount of target nucleic acid initially present in the sample, i.e. the greater the abundance of the target nucleic acid, the smaller the Ct value.

The level of prominin 1-encoding RNA measured in the EVs population may be compared to the expression levels of reference genes in the same vesicles population, such as *e.g.* the beta-actin housekeeping gene. In one embodiment of the invention, levels of prominin 1-encoding RNA in the EVs population measured as Ct value of Ct ≤ 4 in respect to beta-actin mRNA is indicative of renal glomerular disease or of progression of renal glomerular disease (average Ct levels in normal EVs are the followings: beta-actin: 22±2; Prom1: 26±4).

In the context of the present invention, the biological fluid is preferably selected from the group consisting of whole blood, serum, plasma and urine, more preferably the biological fluid is urine. The biological fluid sample may optionally include further components, such as for example: diluents, preservatives, stabilizing agents and/or buffers. If needed, dilutions of the biological fluid sample are prepared using any suitable diluent buffer known in the art.

In one embodiment, the kidney disease which is detected with the method of the invention is an acute glomerular disease, preferably acute post-infectious glomerulonephritis, IgA nephropathy, rapidly progressive glomerulonephritis, membranoproliferative glomerulonephritis and Henoch-Schonlein purpura.

In another embodiment of the method of the invention, the kidney disease is a chronic glomerular disease. Exemplary chronic glomerular diseases include, but are not limited to, diabetic nephropathy, chronic glomerulonephritis, polycystic kidney disease, Alport syndrome and lupus nephritis.

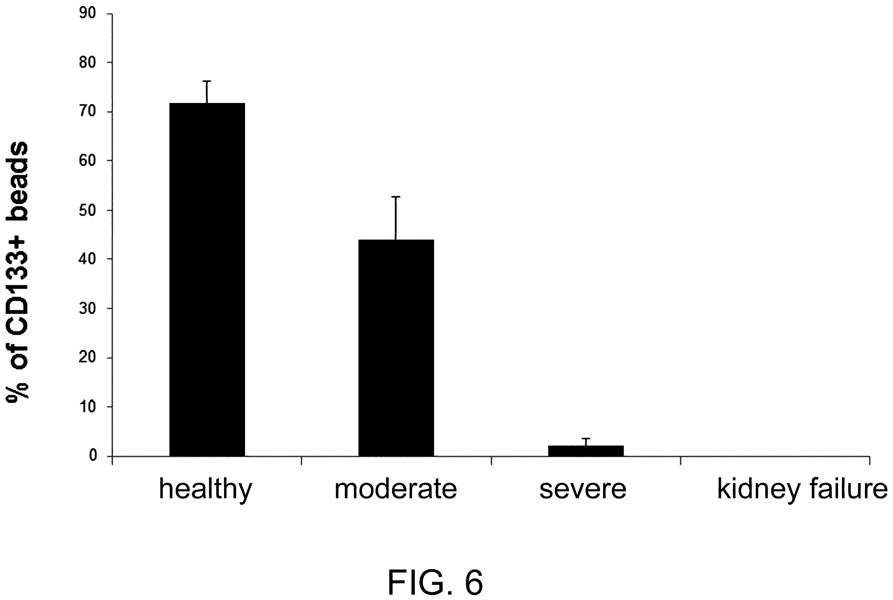

As illustrated in Figure 6, by analyzing biological fluid samples of patients with different stages of renal glomerular disease the present inventors found that a progressive decrease in the level of Prom1⁺ EVs highly correlates with disease worsening.

There is thus provided a diagnostic method that enables to assess the stage of kidney damage as well as to monitor the course of renal glomerular disease. In the context of the present invention, the expression "worsening of renal glomerular disease" is defined as a process of the disease from moderate to severe kidney disease towards end-stage renal disease (kidney failure) requiring renal replacement.

For monitoring the progression of renal glomerular disease, the invention involves comparing the amount of EVs expressing prominin-1 for the biological fluid sample from a subject with a predetermined threshold value.

According to one embodiment, an amount of EVs expressing prominin-1 which is below a predetermined threshold value comprised within the range of from 15% to 40%, for example 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% or 40%, over the entire EVs population, is indicative of moderate glomerular damage.

According to another embodiment of the invention, an amount of Prom1⁺ EVs which is below a predetermined threshold value comprised within the range of from 7% to < 15%, for example 7%, 8%, 9%, 10%, 11%, 12%, 13% or 14%, over the entire EVs population, is indicative of severe glomerular damage.

In a still another embodiment, at the kidney failure stage, the levels of Prom1⁺ EVs are lower than 7%, but not zero, for example 1%, 2%, 3%, 4%, 5% or 6%, over the entire EVs population.

In all the above-described embodiments, the description of a range should be considered to have specifically disclosed all the possible subranges as well as the individual numerical values within that range.

It should be appreciated that the amount of EVs expressing prominin-1 may be assayed in biological samples obtained from a patient at different time points in order to determine whether the amount of Prom1⁺ EVs is decreasing in the patient. Based on the progression of the disease, certain therapeutic decisions may be recommended.

According to the invention, the progression of renal glomerular disease in response to a therapeutic treatment may be monitored. In this context, the method of the present invention may be applied at one or more times after the start of a therapy and the results may be compared to each other or to the result before the start of the therapy in order to assess the success of the therapy.

The invention will be better understood from the following examples which are provided by way of illustration only and which make reference to the appended drawings, wherein:

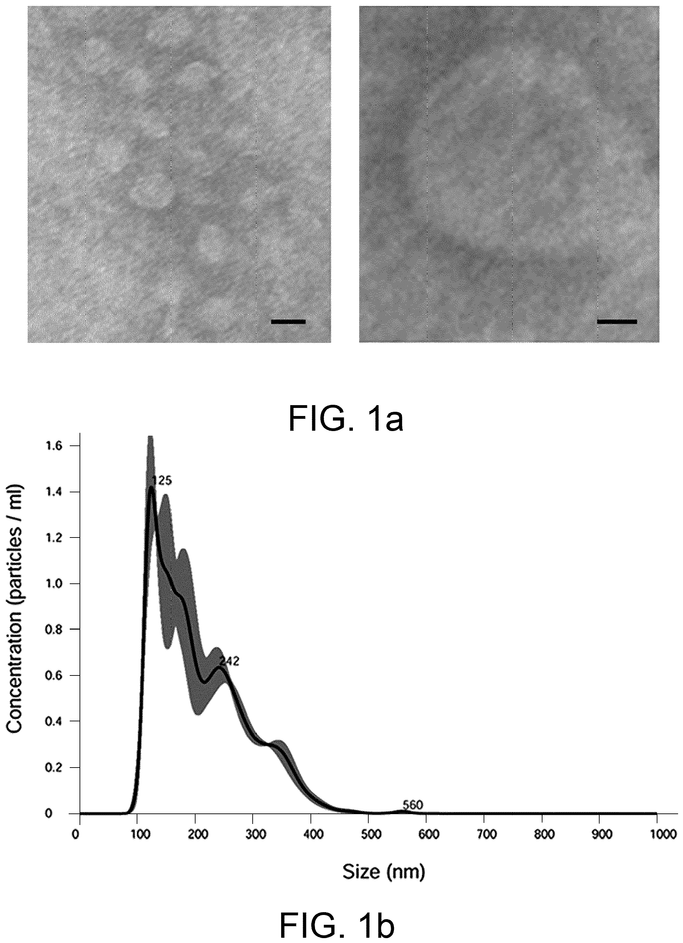

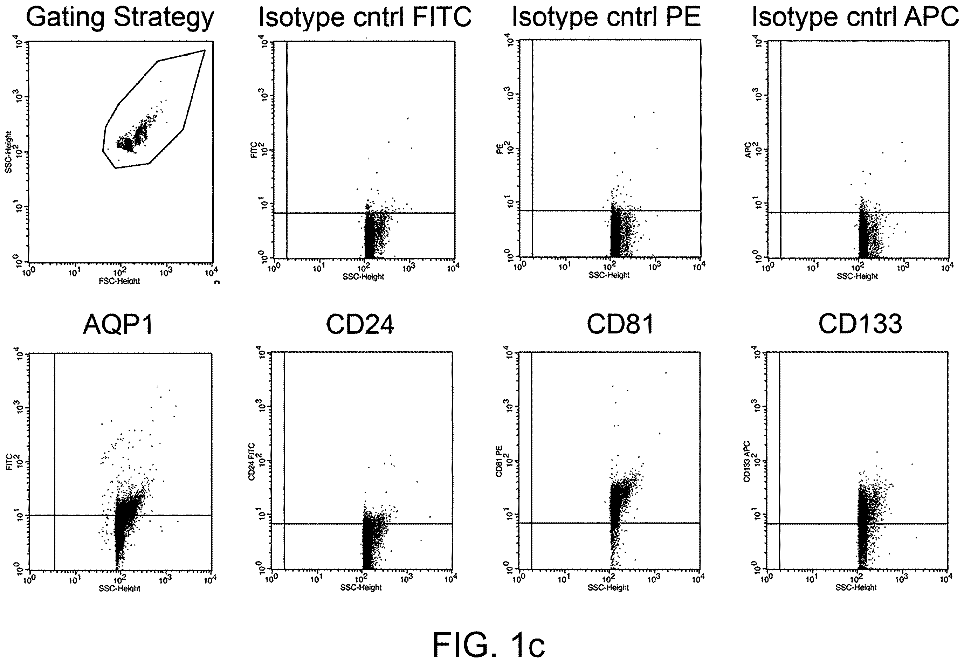

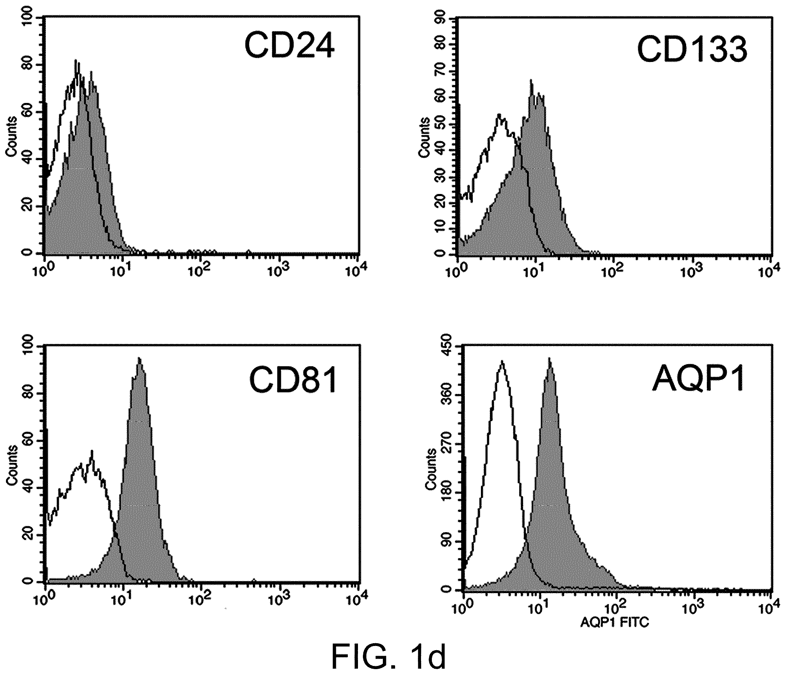

Figure 1 shows the characterization of urinary EVs (uEVs) from healthy subjects. (a) Transmission Electron Microscopy (TEM) image of uEVs isolated from healthy subjects (scale bar 150nm), and an enlargement on one single uEV (scale bar 20nm). (b) Nanoparticle Tracking Analysis (NTA) of a representative pool of uEVs showing their size distribution profile. Cytofluorimetric analysis of surface markers present in healthy subjects uEVs represented both (c) in dot plot (the gating strategy and the negative fluorescence controls are reported) and (d) in histograms with isotype control (black line) plotted on the marker signal (filled line). Ten different preparations were tested with similar results.

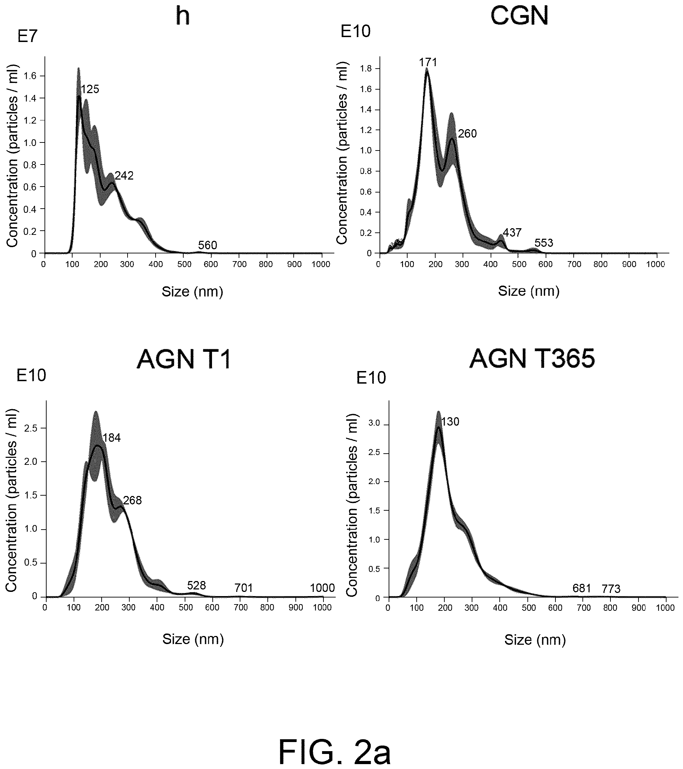

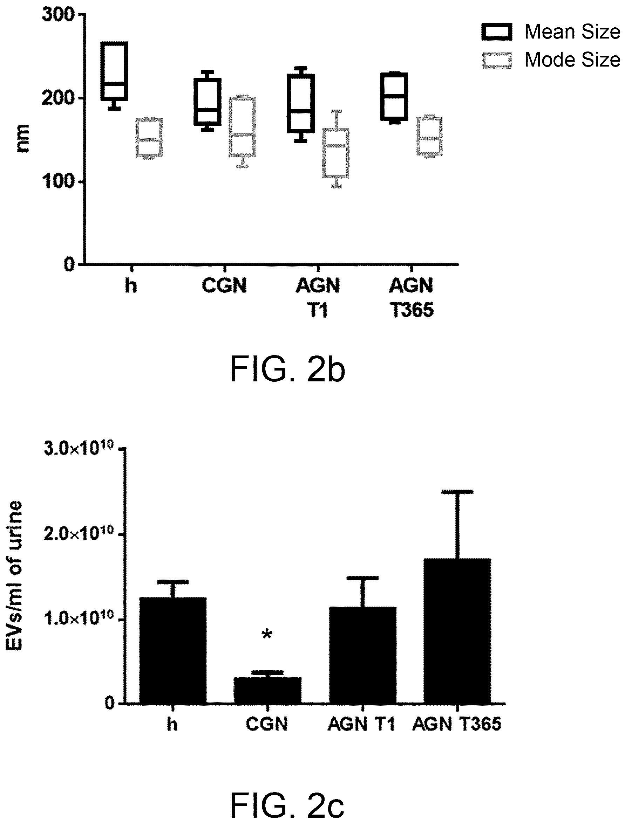

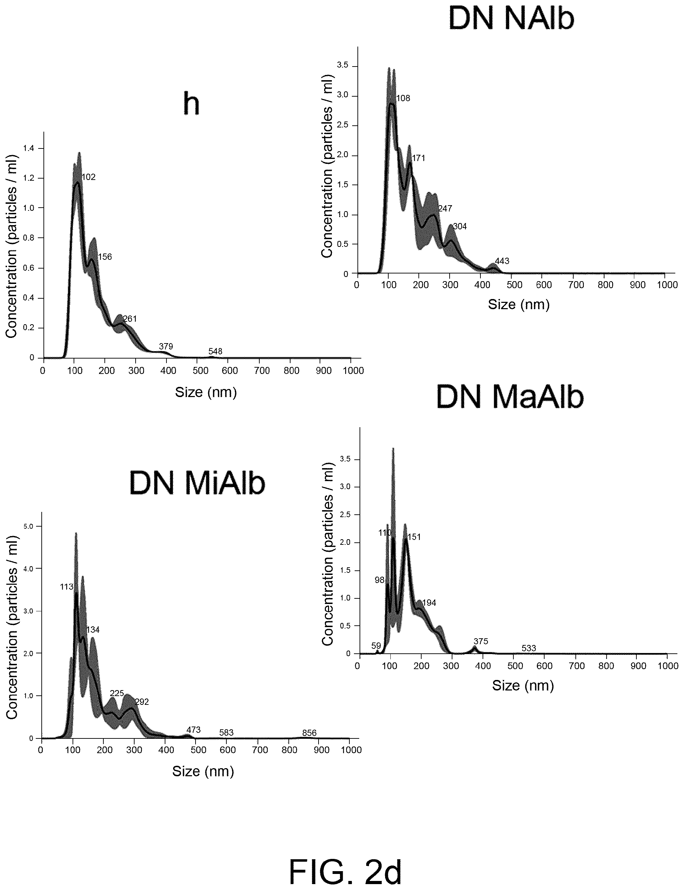

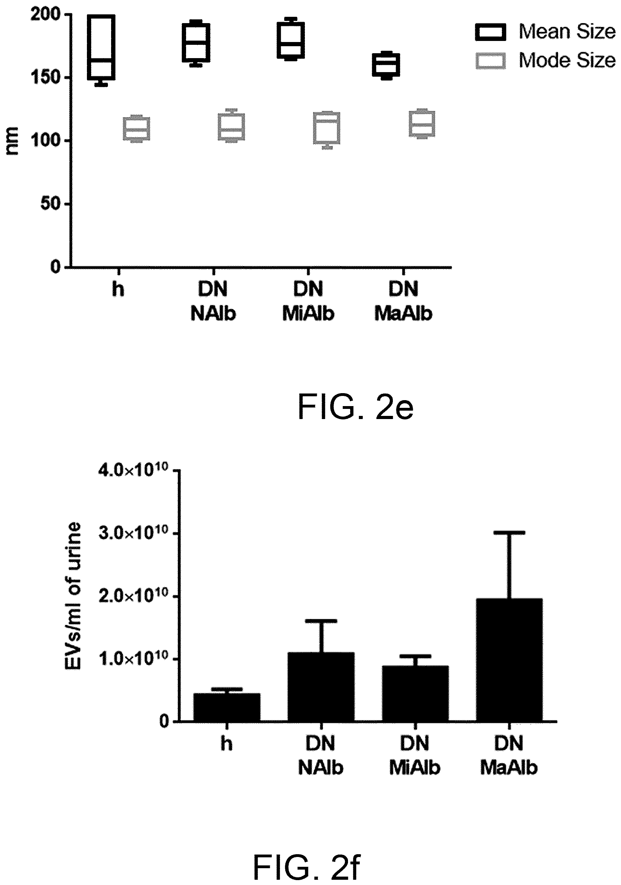

Figure 2 shows the characterization of uEVs from acute and chronic glomerular damaged patients. (a) Nanoparticle Tracking Analysis (NTA) of a representative sample of uEVs coming from each group of paediatric patients. (b) Nanoparticle Tracking Analysis (NTA) of EVs size (nm) in the different groups of paediatric patients, reported both in Mean and Mode values. (c) Evaluation of EVs concentration in the urine of the different groups of paediatric patients. (d) Nanoparticle Tracking Analysis (NTA) of a representative sample of uEVs coming from each group of diabetic patients. (e) Nanoparticle Tracking Analysis (NTA) of EVs size (nm) in the different groups of diabetic patients, reported both in Mean and Mode values. (f) Evaluation of EVs concentration in the urine of the different groups of diabetic patients. Data are expressed as mean ± SE. (*) p<0.05 with unpaired t-test. h: healthy; CGN: chronic glomerulonephritic patients; AGN: acute glomerulonephritic patients one day (T1) and one year (T365) after the damage; NAlb: diabetic normoalbuminuric patients, MiAlb: diabetic microalbuminuric patients; MaAlb: diabetic macro albuminuric patients

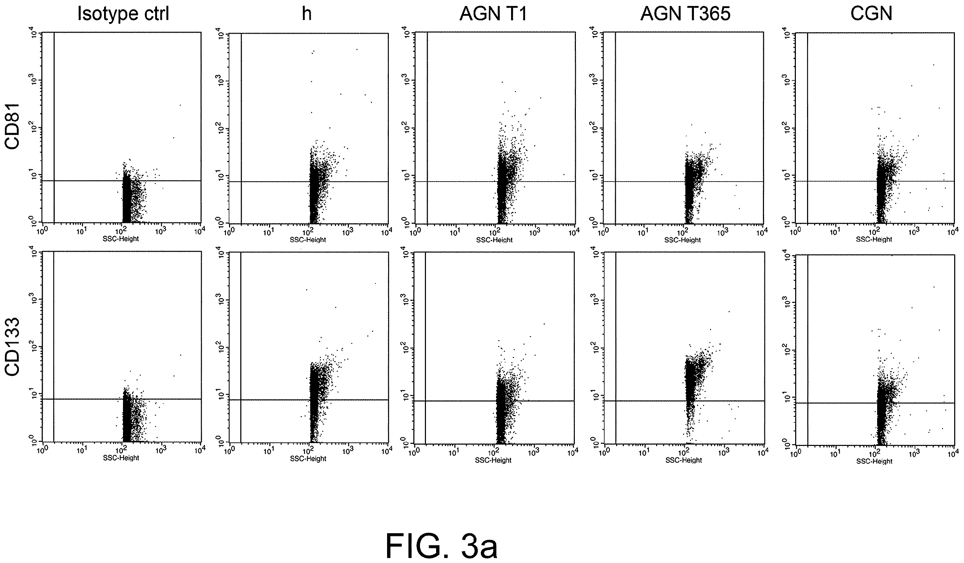

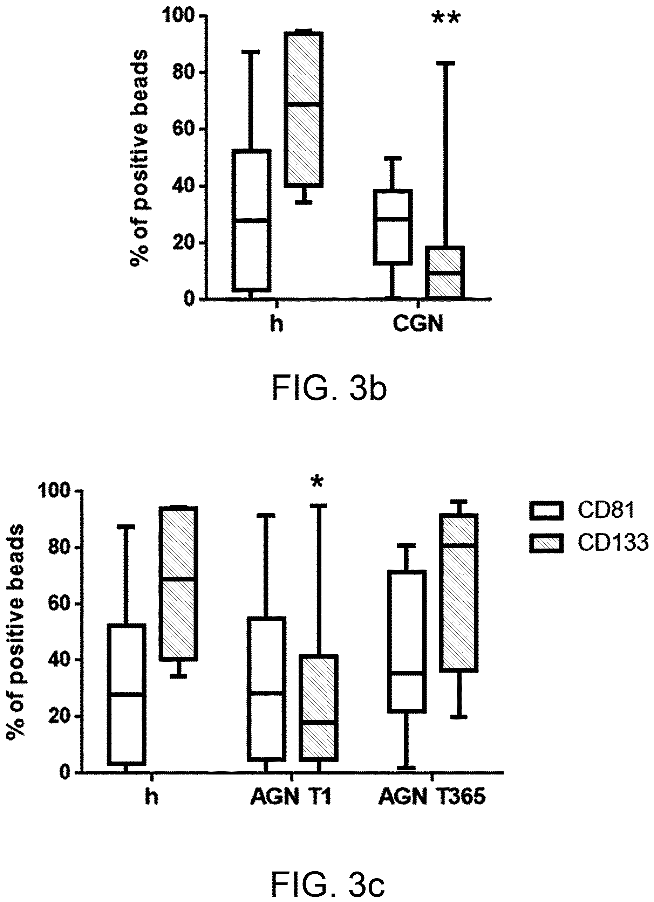

Figure 3 shows uEVs characterization of acute damaged patients. (a) Dot plot cytofluorimetric analysis of surface markers present in uEVs of healthy subjects and in the other groups of paediatric patients. The gating strategy and the negative fluorescence controls are also reported. Box plot representation of cytofluorimetric values of CD81 (black plot) and CD133 (grey filled plot) percentage (b) in the comparison of healthy (h) and chronic glomerulonephritic (CGN) patients, and (c) in the comparison of healthy (h) and acute glomerulonephritic (AGN) patients one day (T1) and one year (T365) after the damage. (d) ROC analysis for the detection of renal damage in different groups of acutely damaged patients using CD133. A table with the CD133 threshold value, the AUC value, the p-value and the odd ratios with confidence interval is also reported.

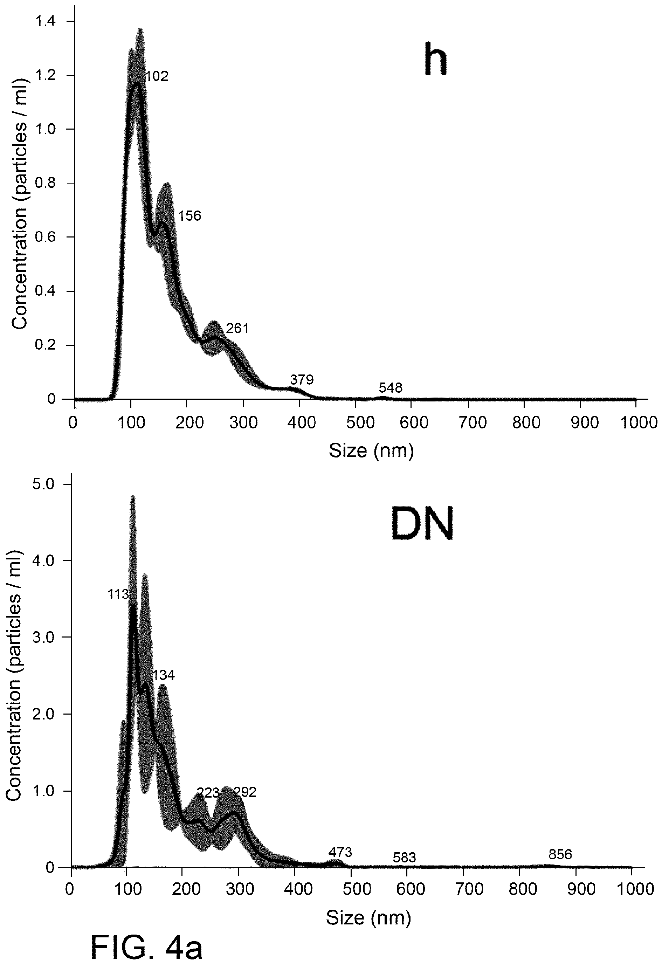

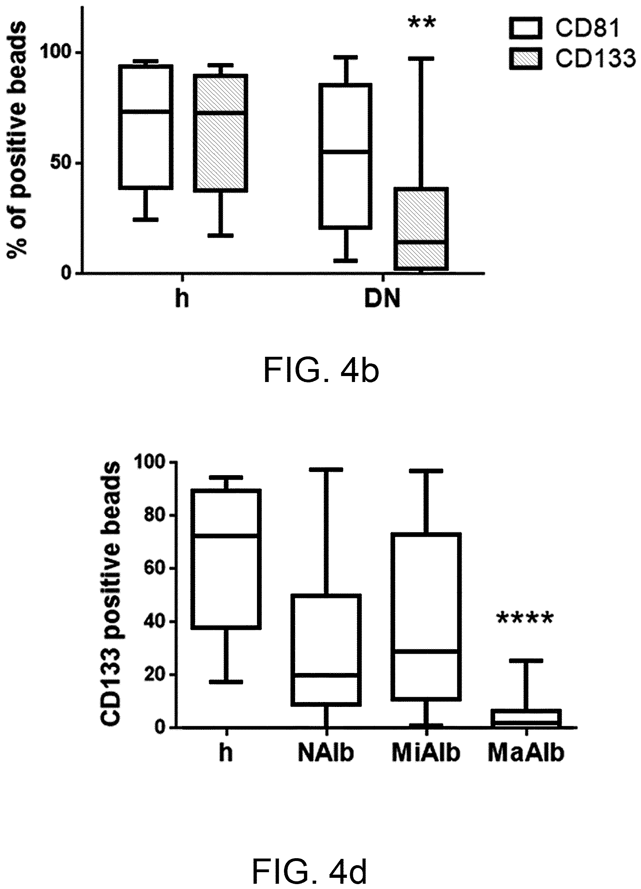

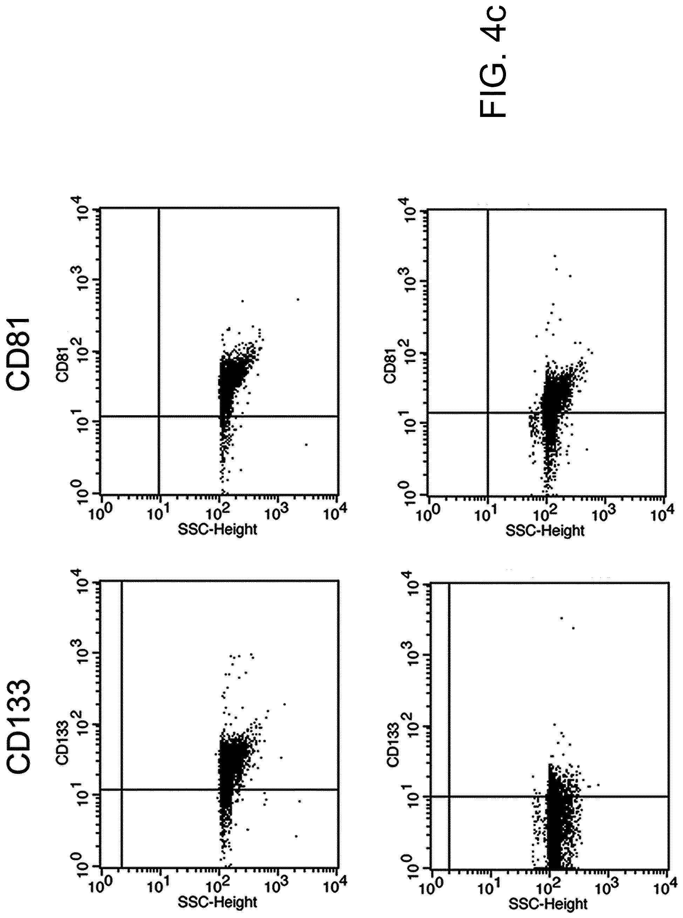

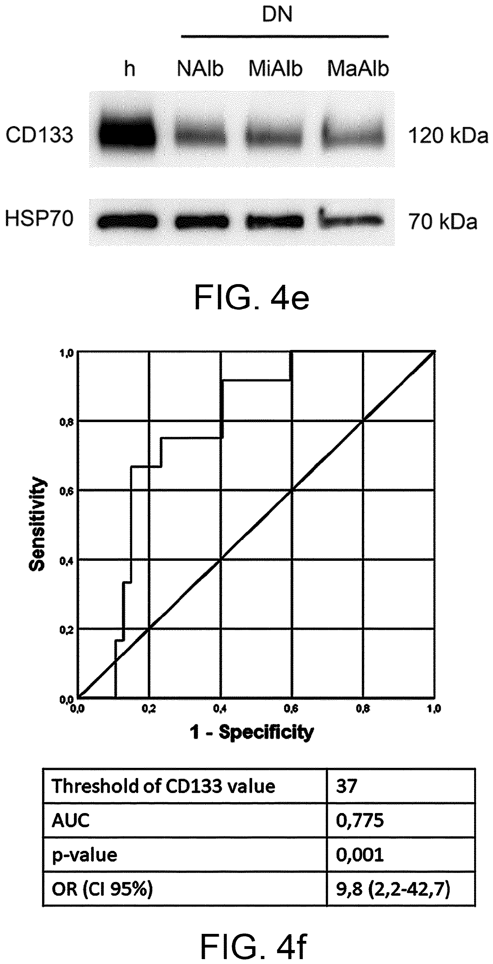

Figure 4 shows uEVs characterization of chronic damaged patients. (A) Nanoparticle Tracking Analysis (NTA) of a representative sample of uEVs coming from healthy (h) and diabetic patients (DN). Cytofluorimetric results reported as dot plot (c) and box plot (b) of CD81 (black plot) and CD 133 (grey filled plot) in healthy (h) and diabetic patients (DN). (d) Cytofluorimetric evaluation of CD133 in uEVs of healthy (h), diabetic normoalbuminuric (NAlb), diabetic microalbuminuric (MiAlb) and diabetic macroalbuminuric (MaAlb) patients. (****) p<0.0001, with Kruskal-Wallis multiple comparison test. (e) Western blot results of CD133 evaluation in floating purified uEVs. (f) ROC analysis for the detection of renal damage in different groups of diabetic patients using CD133. A table with the CD133 threshold value, the AUC value, the p-value and the odd ratios with confidence interval is also reported.

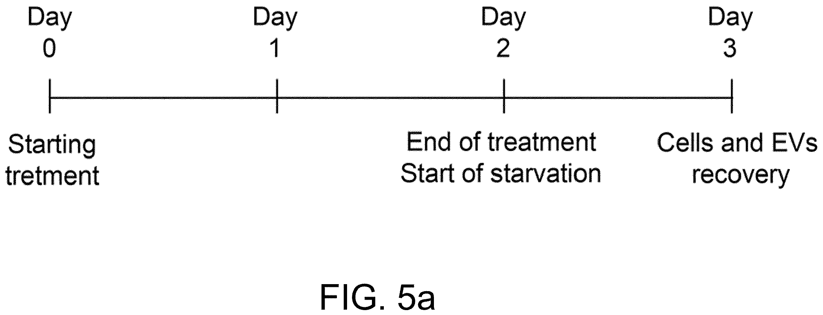

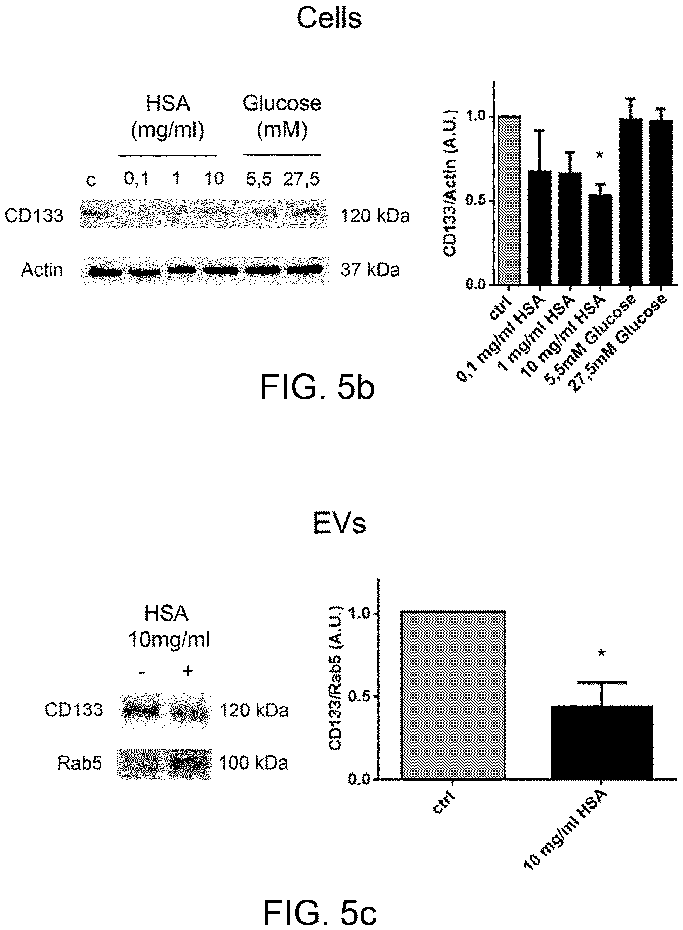

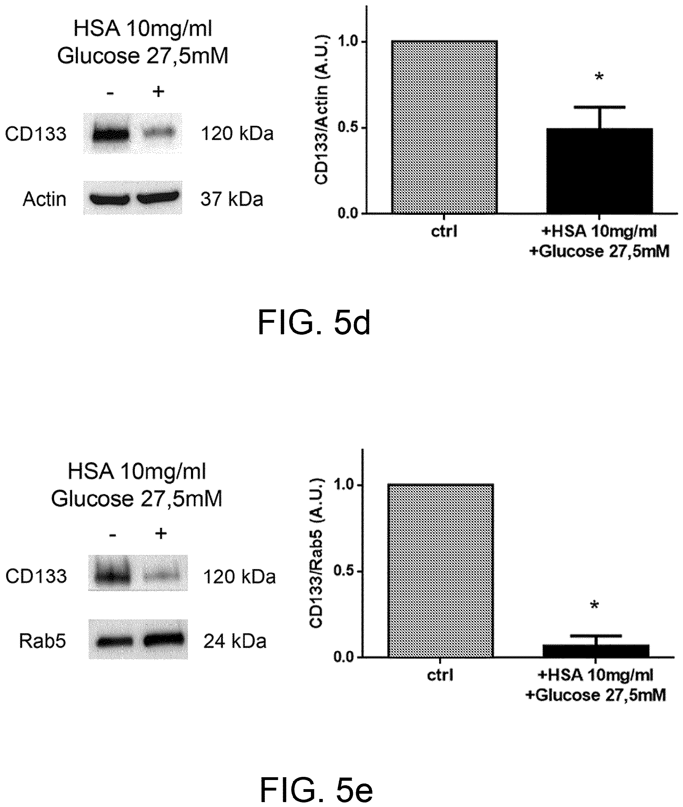

Figure 5 shows that CD133 decreases upon HAS and Glucose treatment. (a) Timetable of the experiment performed on CD133⁺ renal cells. (b) CD133 decrease in CD133⁺ cells upon treatment with increasing HSA concentration (0.1-1-10 mg/ml) but not with Glucose (3.5 - 27.5 mM) treatment. (c) CD133 decrease in CD133⁺ cells derived EVs upon high HAS concentration (10 mg/ml). (d) CD133 decrease in CD133⁺ cells upon combined treatment with HSA 10 mg/ml and Glucose 27.5 mM. (e) CD133 decrease in CD133⁺ cells derived EVs upon combined treatment with HSA 10 mg/ml and Glucose 27.5 mM. Data are expressed as mean ± SE. (*) p<0.05, with unpaired t-test.
Figure 6 is a graph showing the data obtained by measuring the level of CD133⁺ uEVs in urine samples from patients affected by progressive stages of renal glomerular disease (healthy; moderate glomerular damage; severe glomerular damage; kidney failure).

### 1. MATERIAL AND METHODS

### 1.1 Study Groups

All the patients enrolled in the present study provided informed written consent for the study. The study protocol was approved by the Bioethics Committee of the A.O.U. Città della Salute e della Scienza Hospital (Protocol number: 0021671). The study was conducted according to the principles expressed by the Declaration of Helsinki of 1975, as revised in 2013. The study group was composed by a total of 30 paediatric patients and 7 paediatric healthy control subjects. Urine was collected from 18 paediatric poststreptococcal GN patients (Table 1) at time of diagnosis and before any treatment start. At time of resampling (1 year), patients were all treatment-free and experienced no relapse of proteinuria and/or haematuria after first remission. Samples where also collected from 12 CGN paediatric patients (Table 1) with a continuous active proteinuria and/or haematuria for at least 12 months. All patients were under minimum steroid treatment at the time of sampling without additive immunosuppressive drugs.

The validation group was composed by 47 adult type 2 diabetic patients admitted to the Clinic (HbA1c > 48 mmol/mol) and 13 adult healthy subjects matched for age and sex.

### 1.2 Urine collection and EV isolation

Morning urine samples (around 100 ml) were collected from study subjects in sterile containers. In parallel, biochemical analysis were performed by the clinical laboratory of the A.O.U. Città della Salute e della Scienza Hospital. Urine samples were centrifuged at 3000 rpm for 15 min to remove whole cells, large membrane fragments and other debris. Protease Inhibitor (PI) Cocktail (Sigma-Aldrich, Missouri, USA; 100µl PI / 100ml urine) and NaN₃ 10 mM (Sigma-Aldrich) were added immediately to the remaining supernatant. After filtration through 0.8 µm and 0.45 µm filters (Merck Millipore, Burlinghton, MA, USA), uEVs were collected from the samples through an ultracentrifugation (Beckman Coulter, OPTIMA™ L-100 K Ultracentrifuge, Rotor Type 70-Ti Brea, California, USA) at 100,000g for 1 hour at 4°C. The pellet was then resuspended in RPMI (Euroclone, Turin, Italy) + 1% DMSO (Sigma-Aldrich) and stored at -80°C until use.

### 1.3 EV quantification

EVs were quantified by nanoparticle tracking analysis (NTA), using the NanoSight NS300 system (NanoSight Ltd, Salisbury, UK), configured with a Blue 488 nm laser and a high sensitivity digital camera system OrcaFlash 2.8, Hamamatsu C1 1440 (NanoSight Ltd). Briefly, EVs stored in -80°C were thawed, strongly vortexed and properly diluted in physiologic solution (Fresenius Kabi Bad Homburg, Germany) previously filtered with 0.1 µm filter (Merck Millipore). For each sample, three videos of 30-second duration were recorded. The settings of acquisition and analysis were optimized and kept constant between samples.

### 1.4 Transmission Electron Microscopy

Transmission electron microscopy was performed using a Jeol JEM 1010 electron microscope (Jeol, Tokyo, Japan) on uEVs coated on 200 mesh nickel formvar carbon-coated grids (Electron Microscopy Science, Hatfield, PA, USA). EVs were placed in adhesion for 20 minutes on the grids that were then incubated with 2.5 % glutaraldehyde containing 2 % sucrose. After washings in distilled water, EVs were negatively stained with NanoVan (Nanoprobes, Yaphank, NK, USA) and analysed.

### 1.5 Cytofluorimetric analysis

EVs were bound to surfactant-free white aldehyde/sulfate latex beads 4% w/v, 4 µm diameter (Molecular Probes, Thermo Fisher, Waltham, MA, USA) for the subsequent cytofluorimetric analysis. Thirty µg of EVs were incubated with five µl of beads for 30 minutes at room temperature and subsequently for 30 minutes at +4°C. Adsorbed EVs were put in several vials and incubated with the following antibodies: anti-CD133, anti-CD24, anti-CD63, anti-CD81 (Miltenyi Biotech), anti-aquaporin, anti-actin, anti- HSP70/HSC70, anti-Rab5 (Santa Cruz Biotechnology), with a final dilution of 1:50, for 15 minutes at +4°C. The adsorbed EVs were then washed and analyzed with a FACSCalibur and CellQuest software (Becton Dickinson Bioscience Pharmingen, Franklyn Lake, NJ, USA). The anti-Aquaporin-1 antibody (AQP1) (Santa Cruz Biotechnology, Dallas, Texas, USA) was conjugated to an Alexa Fluor® 488 dye through the Alexa Fuor® Antibody Labeling Kit (Molecular Probes) following the manufacturer's instructions. During the cytofluorimetric acquisition the gating strategy was set on the physical parameters dot plot. Controls were represented by EVs adsorbed on beads and marked with FITC-, PE- or APC- conjugated Mouse IgG1 Isotypes (all purchased by Miltenyi, Bergisch Gladbach, Germany).

### 1.6 uEVs floating purification

To avoid protein contamination and increase the purity of uEVs, a floating protocol through a sucrose gradient was applied by the present inventors as described previously (Colombo M, et al. "Biogenesis, Secretion, and Intercellular Interactions of Exosomes and Other Extracellular Vesicles". Annu. Rev. Cell Dev. Biol. (2014); 30:255-89. doi: 10.1146/annurev-cellbio-101512-122326; Kowal J, et al. "Proteomic comparison defines novel markers to characterize heterogeneous populations of extracellular vesicle subtypes". Proc. Natl. Acad. Sci. (2016); 113(8):E968-77. doi: 10.1073/pnas.1521230113). Briefly, uEVs were washed with an ultracentrifuge and resuspended in 1.35 ml of buffer (0.25 M sucrose, 10 mM Tris pH 8 and 1 mM EDTA, all products purchased by Sigma-Aldrich), transferred to a SW55Ti rotor tube (Beckman Coulter) and mixed with 60% stock solution of Optiprep (Sigma-Aldrich) in a 1:1 ratio. Next, 1.2 ml of 20% Optiprep solution was layered on top, followed by 1.1 ml of 10% Optiprep solution. The tubes were then ultracentrifuged at 350,000 g for 1 hour at +4°C with "no brake" deceleration. The next day, five fractions of 1 ml each were collected from the top of the tubes, each fraction was diluted in 25 ml PBS (Lonza, Basel, Switzerland) and washed with an ultracentrifugation at 100,000 g for 2 hours at +4°C to pellet the EVs. The highest fraction, containing pure EVs, as assessed by NanoSight analysis, was then resuspended in 200 µl of DMEM (Lonza) without serum, added with 1% P/S and 1% DMSO (both products purchased by Sigma-Aldrich) to allow freezing storage in -80°C until use.

### 1.7 Cell culture

CD133⁺ Renal Progenitor Cells (RPCs) were obtained from biopsies of normal tissue of a human surgically removed kidney for polar carcinoma performed (after the approval of ethical committee for the use of human tissue of Molinette Hospital; n. 168/2004), as previously described (Brossa A, et al. "Role of CD133 Molecule in Wnt Response and Renal Repair". Stem Cells Transl. Med. (2018); 7(3):283-294. doi: 10.1002/sctm.17-0158.). In particular, the outer medullary tissue at the opposite pole of the tumor was used. The absence of tumor infiltrating cells was evaluated by pathologists. Tissue samples were cut to obtain 3-5 mm3 fragments, digested in 0.1% collagenase type I (Sigma-Aldrich) for 30 min at 37°C and subsequently forced through a graded series of meshes for the separation of cell components from stroma and aggregates. The filtrate was then pelleted by centrifugation. To recover CD133⁺ cells, the single cell suspension underwent magnetic separation for CD133/1 antigen (Miltenyi). CD133⁺ cells (>98% as evaluated by cytofluorimetric analysis) were resuspended in expansion medium (Endothelial Cell Growth Basal Medium plus supplement kit, Lonza) and plated at density 1x10⁴ viable cells per cm². No test for mycoplasma was performed as cells didn't become contaminated.

From passages 2 to 4 cells were treated for 48 hours with Human Serum Albumin (HSA) (Kedrion, Wilmington, DE, USA) at different concentrations (0.1, 1 and 10 mg/ml), Glucose (Sigma Aldrich) at different concentrations (5.5 and 27.5 mM) or a combination of HSA 10 mg/ml plus Glucose 27.5 mM. After 48hours of treatment cells were starved overnight and the next day EVs were recovered from the medium by ultracentrifugation (2 hours, 100,000 g at +4°C).

### 1.8 Western Blot

Proteins from cells were extracted using RIPA buffer, containing 1% of Phosphatase Inhibitor Cocktails, 1% Protease Inhibitors and 1% PMSF (all purchased by Sigma-Aldrich), while proteins from EVs were extracted using the same lysis buffer on a ultracentrifuge pellet. Total protein concentration was estimated by Bradford (Bio-Rad, Hercules, CA) quantification. Ten µg of EV-lysates and 50 µg of cells lysates were loaded on Mini-PROTEAN TGX pre-cast electrophoresis gels (Bio-Rad). Proteins were subsequently transferred on iBlot nitrocellulose membranes (Invitrogen, Carlsbad, California, US) and blotted with antibodies against CD133 (Miltenyi), Actin (Santa Cruz), HSP70/HSC70 (Santa Cruz), Rab5 (Santa Cruz). Chemiluminescent signal was detected using the ECL substrate (Bio-Rad).

### 1.9 Statistical analysis

The expression of different patient data followed a non-normal distribution, as assessed by Kolmogorov-Smirnov test, therefore non parametric tests, such as Mann-Whitney or Kruskal-Wallis, were performed. Western blot experiments were analysed with student's t-test and with ordinary one-way ANOVA with Dunnett's multicomparison test. A p-value < 0.05 was considered significant.

Sensitivity and specificity of CD133 as marker of renal damage, and 95% confidence intervals (95% CI) were calculated using receiver operating characteristic (ROC) curves. The accuracy of the test among different groups of patients was done evaluating areas under curve (AUC) and p-values.

### 2. RESULTS

### 2.1 Characterization of uEVs from healthy paediatric subjects

EVs were isolated from urine obtained from paediatric healthy subjects using a protocol based on filtration and sequential centrifugations. Transmission electron microscopy (TEM) images showed a heterogeneous population of rounded in shape vesicles (Fig. 1a). uEV were also analysed for number and size by Nanosight (mean diameter 265.1±69nm) (Fig. 1b).

Cytofluorimetric analysis was performed using uEV adsorption on latex beads, as this method allows the analysis of small vesicles that cannot be analysed using direct cytofluorimetric analysis. In Figure 1c is shown the gating strategy of the evaluated beads and the setting of the negative threshold. The results showed that uEVs from paediatric healthy subjects, similarly to those obtained from adult healthy subjects, were positive for the renal markers CD24 and AQ1, for the exosomal marker CD81 and for the progenitor/stem cell marker CD133. Urinary EVs did not express the vascular marker VEGF receptor 2 or the podocyte marker podocalyxin (PDX). As control, beads were incubated with antibodies in the absence of EVs and they did not show positive fluorescence.

### 2.2 Characterization of uEVs from paediatric patients with acute and chronic GN

In this study, uEVs from four different groups of patients suffering of glomerulonephritis were compared: healthy subjects, chronic glomerulonephritis (CGN), acute post-infective glomerulonephritis (AGN), either at hospitalization (AGN T1) and on day 365, corresponding to the recovery phase (AGN T365). The Nanosight analysis showed no significant differences in the mean and mode size of the uEVs among the four groups (Fig. 2, a-b). At variance, EV count revealed that the amount of uEVs per ml of urine was significantly decreased in CGN patients compared to healthy subjects (Fig. 2c).

Cytofluorimetric analysis was performed to evaluate the relative amount of uEVs positive for CD133 and CD81, testing the same quantity of EVs previously shown to saturate the latex beads surface (Dimuccio V, et al. "Urinary CD133+ extracellular vesicles are decreased in kidney transplanted patients with slow graft function and vascular damage". PLoS One (2014); 9(8): e104490. doi: 10.1371/journal.pone.0104490). Indeed, as shown in Figure 3, the exosomal marker CD81 did not significantly vary among the different groups, indicating an equal EVs loading. At variance, we observed a significant decrease of CD133 in uEVs of CGN patients compared to healthy subjects. Moreover, CD133⁺ uEVs were also significantly decreased in AGN patients during the acute phase of the disease, to return to basal levels on day 365. All these results are reported in dot plots graphs (Fig. 3a) and in the relative box plots (Fig. 3, c-d). These data indicate that CD133⁺ uEVs, mainly deriving from renal tubular cells are decreased after glomerular damage in both acute and chronic conditions.

Moreover, receiver operating characteristic (ROC) curves were performed, using the values determined by the cytofluorimetric analysis, to investigate the potential utility of CD133 as predictive biomarker of renal damage. By comparing healthy subjects to acute glomerulonephritis patients at day1 (AGN T1) the area under the curve (AUC) value for CD133 was 0.878, with an OR (CI 95%) of 19.5 (3-126.5) and p = 0.001 (Fig. 3d). In the context of this analysis the percentage of CD133⁺ uEVs considered as threshold able to discriminate healthy subjects and acutely damaged glomerulonephritis patients resulted to be 36.

### 2.3 Characterization of uEVs from diabetic patients

With the aim to validate the reduction of CD133⁺ uEVs observed after glomerular damage in paediatric patients, a study was conducted by the inventors in another group of patients affected by a glomerular disease associated to T2D. Based on the degree of their renal impairment, patients were divided in three groups: normoalbuminuric diabetic patients (NAlb DN), microalbuminuric diabetic patients (MiAlb DN) and macroalbuminuric diabetic patients (MaAlb DN), and were compared to age matched healthy subjects. Nanosight analysis showed that the mean and mode uEV dimension are similar among groups. Similarly, no differences in the number of uEVs /ml of urine were detected (Fig. 2, d-f).

The presence of CD133⁺ uEVs in diabetic patients was subsequently compared to that of healthy subjects by cytofluorimetric analysis. In Figure 4b-c the level of CD81 was confirmed constant between healthy and diabetic patients, while CD133⁺ uEVs was significantly decreased in DN patients. Moreover, when the analysis of CD133⁺ uEVs on diabetic patients was based on their albuminuria level, a further decrease of CD133⁺ uEVs was detected in the urine of macroalbuminuric patients (Fig. 4d).

In order to evaluate whether the proteins present in the urine of albuminuric diabetic patients could affect the results of the cytofluorimetric analysis, three pools of uEVs from the three different diabetic groups were purified from possible contaminating proteins by applying a glucose gradient floating protocol (Colombo M, et al. "Biogenesis, Secretion, and Intercellular Interactions of Exosomes and Other Extracellular Vesicles". Annu. Rev. Cell Dev. Biol. (2014); 30:255-89. doi: 10.1146/annurev-cellbio-101512-122326; Kowal J, et al. "Proteomic comparison defines novel markers to characterize heterogeneous populations of extracellular vesicle subtypes". Proc. Natl. Acad. Sci. (2016); 113(8):E968-77. doi: 10.1073/pnas.1521230113). Floated uEVs were analysed in comparison with a pool of healthy uEVs subjected to the same protocol. As reported in Figure 4e the loading of 10 micrograms of proteins from each sample showed a significant decrease in CD133 in all diabetic patients, confirming the results of the cytofluorimetric analysis.

In order to confirm the potential of CD133 as predictive biomarker in renal damage, ROC curves were also performed on the results obtained from cytofluorimetric analysis. By comparing healthy subjects to all diabetic patients the AUC value for CD133 was 0.775, with an OR (CI 95%) of 9.8 (2.2-42.7) and p = 0.001 (Fig. 3d). In the context of this analysis the percentage of CD133+ uEVs considered as threshold able to discriminate healthy subjects and diabetic patients resulted to be 37.

### 2.4 Modulation of CD133⁺ EV release by CD133⁺ cells subjected to Albumin and Glucose overload

To evaluate a possible role of renal progenitor cells in the release of CD133⁺ uEVs, CD133⁺ renal cells were treated with different concentrations of albumin (HSA), or glucose for 48h (Fig. 5a). Western blot analysis showed that CD133⁺ cells lost CD133 expression upon albumin treatment but not upon glucose treatment (Fig. 5b). In parallel, albumin load was also effective in inducing loss of CD133⁺ in CD133⁺ renal cell derived EVs (Fig. 5c). To mimic diabetes in vitro, we treated CD133⁺ cells with high albumin concentration (10 mg/ml) together with high glucose concentration (27.5 mM), for 48 hours. Western blot revealed a strong decrease in CD133 expression both in progenitor renal cells and their derived EVs (Fig. 5, d-e). These data support those observed in patients' urine, and indicate that proteinuria and glucose may modify tubular progenitors cells and promote CD133 loss in cells and in their deriving EVs found in urine.

## Claims

1. An in vitro method of diagnosing or monitoring the progression of renal glomerular disease in a subject comprising the step of determining in a biological fluid sample from the subject the amount of extracellular vesicles (EVs) expressing prominin-1, wherein the amount of said EVs expressing prominin-1 below a predetermined threshold value is indicative of renal glomerular disease or of progression of renal glomerular disease.

2. The method according to claim 1, wherein the predetermined threshold value is comprised within the range of from 35% to 40% of the EVs expressing prominin-1 over the total amount of EVs in the biological fluid sample.

3. The method according to claim 1 or 2, wherein the EVs express the CD133 isoform of prominin-1.

4. The method according to any of claims 1 to 3, wherein the biological fluid is urine.

5. The method according to any of claims 1 to 4, wherein the amount of EVs expressing prominin-1 is determined by cytofluorometry.

6. The method according to any of claims 1 to 4, wherein the amount of EVs expressing prominin-1 is determined by real-time PCR.

7. The method according to any of claims 1 to 6, wherein the renal glomerular disease is acute renal glomerular disease selected from the group consisting of acute, acute post-infectious glomerulonephritis, IgA nephropathy, rapidly progressive glomerulonephritis, membranoproliferative glomerulonephritis and Henoch-Schonlein purpura.

8. The method according to any of claims 1 to 6, wherein the renal glomerular disease is chronic renal glomerular disease selected from the group consisting of diabetic nephropathy, chronic glomerulonephritis, polycystic kidney disease, Alport syndrome and lupus nephritis

9. The method according to claim 8, wherein the renal glomerular disease is moderate glomerular damage and the predetermined threshold value is comprised within the range of from 15% to 40% of the EVs expressing prominin-1 over the total amount of EVs in the biological fluid sample.

10. The method according to claim 8, wherein the renal glomerular disease is severe glomerular damage and the predetermined threshold value is comprised within the range of from 7% to < 15% of the EVs expressing prominin-1 over the total amount of EVs in the biological fluid sample.

11. The method according to claim 8, wherein the renal glomerular disease is kidney failure and the predetermined threshold value is comprised within the range of from > 0% to < 7%.
